# EUROPEAN PATENT APPLICATION

(11) **EP 4 523 601 A1**
(43) Date of publication of application: **19.03.2025**
(21) Application number: 24200024.8
(22) Date of filing: 12.09.2024
(51) Int. Cl.: A61B 1/12, A61B 1/00, A61B 90/70

(54) **METHOD AND ARRANGEMENT FOR PRE-CLEANING AN ENDOSCOPE**

(30) Priority: 13.09.2023 US 202363538111 P
(71) Applicant: Olympus Winter & Ibe GmbH, 22045 Hamburg (DE)
(72) Inventor: Ottens, Benjamin, 22399 Hamburg (DE)
(74) Representative: Eisenführ Speiser

(57) **Abstract**

The invention relates to a method for pre-cleaning an endoscope. Further, the invention relates to an arrangement for pre-cleaning an endoscope. It is provided a method for pre-cleaning an endoscope, preferably immediately or shortly after use of the endoscope, the method comprising: providing an endoscope that comprises channels for transporting fluids, connection ports, in particular for connection of fluid hoses, and a distal end of the endoscope, wherein each of the channels extends from one of the connection ports to the distal end of the endoscope; fluidly connecting the channels to each other at the distal end of the endoscope by arranging a container onto the distal end of the endoscope; connecting a suction device, preferably a suction pump, to one of the connection ports; connecting at least one liquid source to at least one of the connection ports; flushing at least one of the channels with a liquid by activating the suction device so that the liquid is drawn from the liquid source through the at least one channel via the distal end towards the suction device.

## Description

### TECHNICAL FIELD

The invention relates to a method for pre-cleaning an endoscope. Further, the invention relates to an arrangement for pre-cleaning an endoscope.

### BACKGROUND

An endoscope is an inspection instrument that can be used to examine, and optionally manipulate, the inside of organisms. Endoscopes can be used in human medical diagnostics for examinations or minimally invasive surgical procedures on humans or animals. Endoscopes typically comprise an image sensor, an optical lens, and a light source.

After use of an endoscope, the endoscope is typically pre-cleaned, manually or automated cleaned, disinfected, and possibly sterilized before being used again on a patient. If an endoscope and accessories used in the patient procedure are not immediately cleaned after each patient procedure, residual organic debris will begin to dry and solidify, hindering effective removal and reprocessing efficacy. Therefore, it is necessary to pre-clean the endoscope and the accessories at the bedside immediately after each patient procedure.

Typically, the pre-cleaning that is conducted at the bedside immediately after each patient procedure inter alia comprises the following steps: First, a biopsy/suction channel is pre-cleaned by arranging a distal end of the endoscope in water, and drawing the fluid through the endoscope. Then, air is drawn through the endoscope. Second, an air/water channel is pre-cleaned by arranging the distal end of the endoscope in water and flush the air/water channel with the fluid. Then, the air/water channel is flushed with air. Third, if available, an auxiliary water channel is pre-cleaned by flushing the auxiliary water channel with water. Finally, the accessories are detached from the endoscope and placed in a fluid, such as water or a detergent solution. Typically, after the pre-cleaning procedure the endoscope is detached from the equipment (such as for example video system center, light source, suction pump) used in the patient procedure and transported to a reprocessing area.

In US 11445900B2 it was suggested to pre-clean an endoscope by using a plurality of input members in form of flexible tubes for use in delivering cleaning solution to multiple input ports of an endoscope, so that a cleaning solution is delivered through the flexible tubes to the endoscope.

However, known procedures for pre-cleaning are both relatively time-consuming and prone to errors. As the pre-cleaning typically consists of several subsequently conducted manually applied steps, the pre-cleaning is relatively time-consuming and if only one of these steps is not conducted exactly according to the pre-cleaning procedure, the endoscope might not be pre-cleaned sufficiently. Furthermore, because not all endoscope channels are connected to each other it is not possible to flush all channels with only one source. In typically conducted pre-cleaning procedures, the video system center is required to flush the air/water channel, a suction pump to aspire the suction and biopsy channel and a flushing pump or syringe to flush the auxiliary water channel. Therefore, the user is permanently involved in the procedure and needs to interact with several different user interfaces which requires that the user can operate all of these user interfaces. Additionally a lot of equipment/accessories is necessary to conduct the pre-cleaning procedure.

Furthermore, a problem is that after pre-cleaning, drying and solidification can occur in the channels that are no longer filled with fluid.

### SUMMARY

Therefore, it is an object of the present invention to provide an improved method and an improved arrangement for pre-cleaning an endoscope. In particular, it is an object of the present invention to provide a method for pre-cleaning an endoscope that is less prone to errors.

According to a first aspect, it is provided a method for pre-cleaning an endoscope, preferably immediately or shortly after use of the endoscope, the method comprising: providing an endoscope that comprises channels for transporting fluids, connection ports, in particular for connection of fluid hoses, and a distal end of the endoscope, wherein each of the channels extends from one of the connection ports to the distal end of the endoscope; fluidly connecting the channels to each other at the distal end of the endoscope by arranging a container onto the distal end of the endoscope; connecting a suction device, preferably a suction pump, to one of the connection ports; connecting at least one liquid source to at least one of the connection ports; flushing at least one of the channels with a liquid by activating the suction device so that the liquid is drawn from the liquid source through the at least one channel via the distal end towards the suction device.

In the following, initially the method for pre-cleaning an endoscope and its functions and advantageous embodiments will be described.

The distal end of the endoscope may also be referred to as the distal tip of the endoscope. Preferably, the distal end is to be understood as the end portion of the endoscope that is located at the very front of an insert section of the endoscope, in particular the end portion that is first inserted into the patient when the insert section of the endoscope is moved into a patient. Preferably, outlets of the channels are arranged at the distal end. Further preferably, a light guide is arranged at the distal end. Further preferably, an objective lens can be arranged at the distal end. It is particularly preferred if the distal end is arranged at the end of an insert section that has a flexible insertion tube.

Preferably, the method is conducted immediately or shortly after use of the endoscope in a patient procedure. Preferably the method for pre-cleaning the endoscope is started within 60 minutes after being used in the patient procedure, particularly preferably within 30 minutes after being used in the patient procedure, in particular within 15 minutes after being used in the patient procedure. Preferably, the pre-cleaning is conducted at a patient bedside so that the method can be conducted immediately or shortly after use of the endoscope in a patient procedure without the need to move the endoscope to another location prior to conducting the pre-cleaning.

Preferably, at least two of the channels are flushed with a liquid by activating the suction device so that the liquid is drawn from the liquid source through at least one of the channels via the distal end and then from the distal end through at least one other channel of the channels towards the suction device.

Preferably, the channels each extend from one of the connection ports to the distal end of the endoscope. However, the endoscope can also have additional channels that do not extend from one of the connection ports to the distal end.

Preferably, the connection ports are to be understood as ports to which fluid hoses or the like can be connected to.

Preferably, the endoscope can for example be a colonoscope, or a gastroscopes, or an ultrasonic endoscope, or a duodenoscopes, or another type of endoscope.

The container can be arranged onto the distal end in a condition in which it is filled with liquid or in which it is not filled with liquid. If the container is not filled with liquid, a continuous flow of liquid can be drawn through the channels via the distal end.

Preferably, before flushing channels of the endoscope, the container is not filled with liquid. An advantage of such an empty container is that liquid can be drawn from a connection port through a channel to the container and from the container through a suction channel towards the suction device out of the endoscope, wherein no gaps of liquid that contain gas are present. Thus, when liquid is drawn out of a connection port, then the user can be sure that this liquid has been drawn completely through at least two channels. However, alternatively, it is also possible that the container is already filled with liquid before flushing the channels. Filling the container with liquid before starting the flushing of the channels can have the advantage that the distal end of the endoscope is in contact with liquid for a longer period of time and soaked for a longer period of time, which can be beneficial to achieve a more thorough pre-cleaning of the distal end, if needed.

An advantage of this method is that even though not all channels of the endoscope are connected to each other inside of the endoscope, it is possible to flush all of these channels with only one source by connecting the cannels to each other via the container that is arranged onto the distal end of the endoscope. Therefore, the pre-cleaning procedure can be conducted faster, easier and in a way that is less prone to errors.

An advantage of this method is that the channels can be kept humid and/or filled with liquid after having conducted the pre-cleaning procedure. Thus, drying and solidification in the channels after pre-cleaning can be prevented in a particularly advantageous way.

Another advantage of this method is that during conducting the pre-cleaning of the endoscope it is not necessary to interact with several user interfaces and/or devices in order to conduct the pre-cleaning procedure.

Another advantage of this method is that compared to conventional pre-cleaning methods, with the described method, much less equipment and/or accessories is necessary to conduct the pre-cleaning. For example, no video center or flushing pump is necessary to conduct the pre-cleaning.

In a particularly preferred embodiment, connecting at least one liquid source to at least one of the connection ports comprises connecting at least one vessel that holds liquid, preferably at least three, in particular four, vessels that hold liquid, to at least one of the connection ports, preferably to at least three, in particular four, of the connection ports.

An advantage of connecting at least one vessel that holds liquid to at least one of the connection ports is that, in particular by applying an underpressure, the liquid can be drawn from the vessel through the channel that extends from the connection port to which the vessel is connected to the distal end of the endoscope and because the container is arranged at the distal end the liquid can further be drawn from the distal end through another channel, in particular towards a suction device. This way it can be achieved that the liquid is drawn through the entire extension of both of these channels starting from the vessel, through the first channel via the distal end and then through the other channel, so that both of these channels are reliably flushed with the liquid along their whole extension. Further channels can be reliably flushed in the same way. Therefore, it is preferable to arrange vessels onto several connection ports so that all of the channels that extend from these connection ports to the distal end can reliably be flushed with liquid along their whole extension.

In a further preferred embodiment, the method comprises connecting closure elements to each of the connection ports to which no liquid source and no suction device is connected.

It is particularly preferred to connect each of the connection ports either to a vessel or to a closure element or to a suction device. Preferably, then each of the connection ports is connected to a vessel or to a closure element or, preferably indirectly via a hose or the like, to a suction device.

Closure elements are preferably to be understood as elements that are adapted to close a connection port, preferably fluid-tight, in particular air-tight, when being applied onto the connection port. Preferably, closure elements are connected to such connection ports that are connected to a channel that does not need to be flushed in the pre-cleaning procedure.

In a further preferred embodiment, the method comprises flushing at least two of the channels, preferably all of the channels, with liquid by activating the suction device so that liquid is drawn through the channels via the distal end towards the suction device.

By activating the suction device, an underpressure can be obtained with which the liquid can be drawn through the cannels towards the suction device. Preferably, only one of the channels is connected to the suction device.

In a further preferred embodiment, the channels comprise an air channel that extends from an air channel connection port to the distal end of the endoscope, a suction channel that extends from a suction channel connection port to the distal end of the endoscope, a water channel that extends from a water channel connection port to the distal end of the endoscope, and preferably an auxiliary water channel that extends from an auxiliary water channel connection port to the distal end of the endoscope.

Preferably, the air channel is adapted to provide air to the distal end of the endoscope. Preferably, the suction channel is adapted to draw liquids and/or objects, such as for example tissue samples, from the distal end of the endoscope through the endoscope. Preferably, the water channel is adapted to provide water at the distal end of the endoscope. Preferably, the auxiliary water channel is adapted to provide water at the distal end of the endoscope.

Preferably, the channels are arranged parallel to each other over at least a part of their extension from one of the connection ports to the distal end of the endoscope.

In a further preferred embodiment, the container is arranged onto the distal end of the endoscope in such a manner that the distal end, including the outer surface of the distal end, is arranged entirely inside the container.

Preferably, the outer surface, in particular formed as outer circumferential surface, of the distal end is arranged inside of the container when the container is filled with fluid.

In a further preferred embodiment, the container is filled with liquid and the whole distal end, including the outer surface of the distal end, is in contact with the liquid.

The pre-cleaning of the distal end can be challenging, especially if the distal end has a complex geometry, which is for example the case for duodenoscopes. An advantage of arranging the whole distal end, including the outer surface, inside of the container is that and when arranging the whole distal end in the container and filling the container with fluid, the distal end and its outer surface can reliably be pre-cleaned because the liquid then is in contact with all surfaces, including the outer surface, of the distal end.

In a further preferred embodiment, the container is arranged on the distal end of the endoscope in such a manner that the distal end of the endoscope is sealed liquid-tight, and preferably gas-tight, from the environment that surrounds the container.

An advantage of sealing the inside of the container against the environment is that in this way no external fluids can enter the channels and no liquid can flow out from the container in the environment that surrounds the container.

Preferably, the inner volume of the container is not changeable. This can be achieved, for example, by using a relatively stiff material for the wall of the container that defines an inner chamber of the container. This way, when an underpressure is applied to the inner chamber of the container, the inner volume of the container does not contain but remains substantially unchanged so that the fluid connection of the channels at the end of the endoscope remains even when an underpressure is applied to the channel system and thus to the inner volume of the container.

In a further preferred embodiment, each of the connection ports is connected to a vessel, except for the connection port that is connected to the suction device.

An advantage of such a preferred arrangement is that by arranging the vessels to all connection ports except for the connection port that is connected to the suction device all channels can reliably be flushed and thus pre-cleaned. Thus, such an arrangement is particularly preferably if all channels shall be flushed.

In a further preferred embodiment, the suction device is fluidly connected to a suction container that is adapted to receive liquid drawn out of the endoscope by the suction device.

By connecting such a suction container to the suction device, the liquid drawn through the channels and out of the endoscope via the suction device can be safely collected.

In a further preferred embodiment, the liquid that is drawn through at least one of the channels is water or a disinfectant solution or a detergent solution.

It is preferred to use disinfectant solution and/or detergent solution in order to achieve a better removal of organic debris when compared to water.

In a further preferred embodiment, the endoscope is immersed in a liquid so that the connection ports are in contact with the liquid, wherein preferably the connection port that is connected to the suction device is connected to the suction device via a hose or the like.

In such an alternative solution compared to a solution in which vessels are arranged onto connection ports, the endoscope can fully or partly be immersed in a liquid so that the connection ports are in contact with the liquid in which the endoscope is immersed in.

Preferably, the connection port that is connected to the suction device is connected to the suction device via a hose or the like. This connection port can also be immersed in the liquid, but preferably without providing a fluid connection between the channel that is connected to the connection port and the liquid.

According to a further aspect, it is provided an arrangement for pre-cleaning an endoscope, preferably immediately or shortly after use of the endoscope, in particular for pre-cleaning an endoscope according to the method described herein, the arrangement comprising: an endoscope that comprises channels for transporting fluids, connection ports, in particular for connection of fluid hoses, and a distal end of the endoscope, wherein each of the channels extends from one of the connection ports to the distal end of the endoscope; a container that is arranged onto the distal end of the endoscope in such a manner that the channels are fluidly connected to each other at the distal end of the endoscope; at least one liquid source that is connected to at least one of the connection ports; and preferably a suction device, preferably a suction pump, that is connected to one of the connection ports.

The advantages explained above with respect to the method for pre-cleaning are also advantages for such an arrangement for pre-cleaning. The advantages are in particular achieved by arranging the container onto the distal end in such a manner.

In a further preferred embodiment, the suction device is adapted to flush at least one of the channels with a liquid by activating the suction device so that the liquid is drawn from the liquid source through the at least one channel via the distal end towards the suction device.

In a further preferred embodiment, the endoscope comprises: an air channel that extends from an air channel connection port to the distal end of the endoscope, a suction channel that extends from a suction channel connection port to the distal end of the endoscope, a water channel that extends from a water channel connection port to the distal end of the endoscope, and preferably an auxiliary water channel that extends from an auxiliary water channel connection port to the distal end of the endoscope, and preferably at least one closure element that is connected to each of the connection ports to which no liquid source and no suction device is connected.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein.

### BRIEF DESCRIPTION OF THE DRAWINGS

Preferred embodiments shall now be described with reference to the attached drawings, in which
- Fig. 1:: shows a schematic depiction of an exemplary embodiment of an arrangement for pre-cleaning an endoscope;
- Fig. 2:: shows a schematic flow diagram of an exemplary embodiment of a method for pre-cleaning an endoscope;
- Fig. 3:: shows a schematic flow diagram of an exemplary embodiment of a method for pre-cleaning an endoscope.

### DETAILED DESCRIPTION

In the figures, elements with the same or comparable functions are indicated with the same reference numerals.

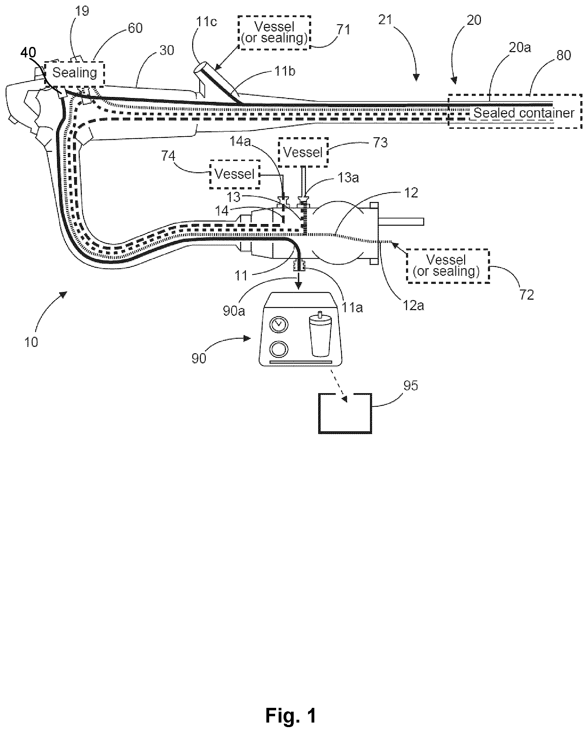

Fig. 1 shows a schematic depiction of an exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a suction channel connection port 11a to the distal end 20 of the endoscope 10. The distal end 20 is the end portion of the endoscope 10 that is located at the very front of an insert section 21 of the endoscope 20.

The air channel 12 is adapted to provide air to the distal end 20 of the endoscope 10. The air channel 12 extends from an air channel connection port 12a to the distal end 20 of the endoscope 10.

The water channel 13 is adapted to provide water at the distal end 20 of the endoscope 10. The water channel 13 extends from a water channel connection port 13a to the distal end 20 of the endoscope 10.

The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements. Each of the channels 11, 12, 13, 14 extends from one of the connection ports 11a, 12a, 13a, 14a to the distal end 20 of the endoscope 10.

The channels 11, 12, 13, 14 are fluidly connected to each other at the distal end 20 of the endoscope 10 by arranging a container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface 20a of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

The endoscope 10 furthermore comprises a biopsy branch 11b that extends from a biopsy branch connection port 11c to the suction channel 11.

The air channel 12 and the water channel 13 can be fluidly connected and/or separated from each other via an air/water cylinder 19. A vent can be assembled to the air/water cylinder 19. The air/water cylinder 19 is sealed with a closure element 60 that is adapted to close the air/water cylinder 19 fluid-tight. Furthermore, a suction cylinder 40 is provided to which the suction channel 11 is connected to. A vent can be assembled to the suction cylinder 40.

A vessel 71 is arranged onto the biopsy branch connection port 11c. If the biopsy branch 11b does not need to be flushed during the pre-cleaning procedure, a closure element can be arranged onto the biopsy branch connection port 11c instead of the vessel 71.

A vessel 72 is arranged onto the connection port 12a of the air channel 12. If the air channel 12 does not need to be flushed during the pre-cleaning procedure, a closure element can be arranged onto the connection port 12a instead of the vessel 72.

A vessel 73 is arranged onto the connection port 13a of the water channel 13. If the water channel 13 does not need to be flushed during the pre-cleaning procedure, a closure element can be arranged onto the connection port 13a instead of the vessel 73.

Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74.

The vessels 71, 72, 73, 74 are filled with liquid, in particular with a disinfectant solution and/or detergent solution.

A suction device 90 that is a suction pump in this preferred embodiment is connected via a fluid hose 90a to the connection port 11a of the suction channel 11. When the suction device 90 is activated, an underpressure is applied to the system so that liquid is drawn towards the suction device 90 as described in detail below.

Liquid is drawn from the vessel 71 through the biopsy branch 11b and from there further through the suction channel 11, out of the connection port 11a, via the fluid hose 90a to the suction device 90 and from the suction device 90 to the suction jar 95.

Liquid is drawn from the vessel 72 through the air channel 12 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 73 through the water channel 13 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 74 through the auxiliary water channel 14 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

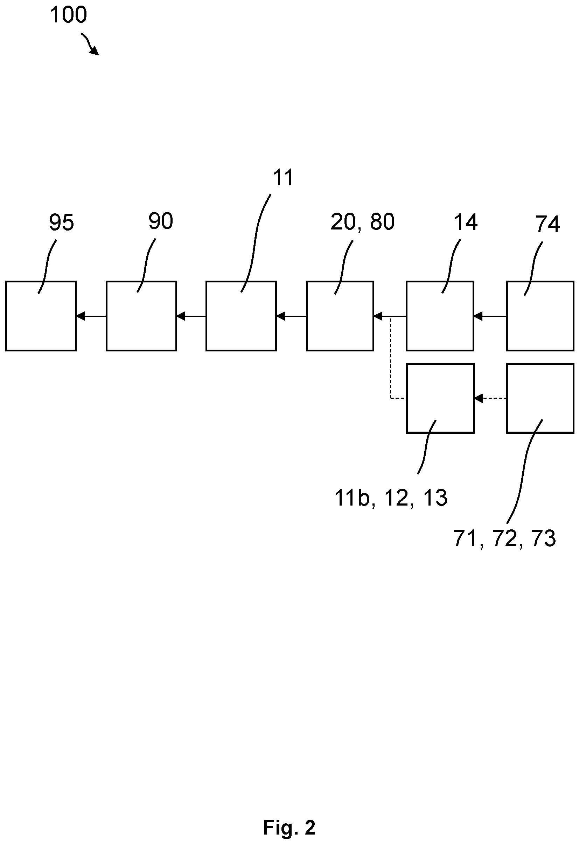

Fig. 2 shows a schematic flow diagram of an exemplary embodiment of a method 100 for pre-cleaning an endoscope 10 that is designed as shown in Fig. 1. The method 100 for pre-cleaning the endoscope 10 comprises fluidly connecting the channels 11, 11b, 12, 13, 14 of the endoscope to each other at the distal end of the endoscope by arranging a container onto the distal end 20 of the endoscope and connecting a suction device 90 to a channel 11, wherein the suction device 90 is further connected to a suction container, formed as a suction jar 95. Vessels 74, 71, 72, 73 that contain liquid are arranged onto the connection ports of the channels 11b, 12, 13, 14. By activating the suction device 90, liquid is drawn in the direction indicated by the arrows, namely from the vessels 74, 71, 72, 73 through the channels 11b, 12, 13, 14 (to which the vessels 74, 71, 72, 73 are connected to) towards the distal end 20 and from the distal end 20 via the container 80 into the suction channel 11 and through the suction channel 11 and through the suction channel to the suction pump 90 to be then collected in the suction jar 95.

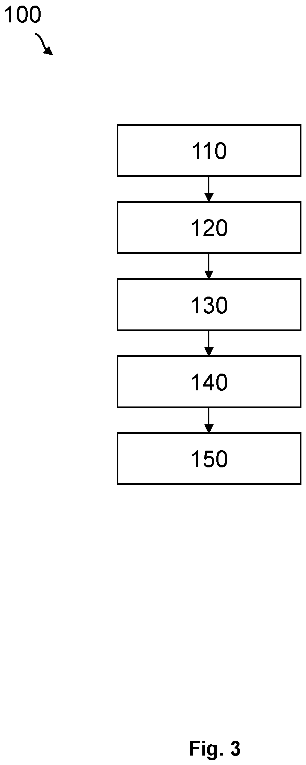

Fig. 3 shows a schematic flow diagram of an exemplary embodiment of a method 100 for pre-cleaning an endoscope as shown and described in connection with Fig. 1. The method 100 is conducted immediately or shortly after use of the endoscope in a patient procedure and comprises the following steps:
In a step 110, providing an endoscope that comprises channels for transporting fluids, connection ports, in particular for connection of fluid hoses, and a distal end of the endoscope, wherein each of the channels extends from one of the connection ports to the distal end of the endoscope.
In a step 120, fluidly connecting the channels to each other at the distal end of the endoscope by arranging a container onto the distal end of the endoscope.
In a step 130, connecting a suction device, preferably a suction pump, to one of the connection ports.
In a step 140, connecting at least one liquid source to at least one of the connection ports.
In a step 150, flushing at least one, preferably at least two, of the channels with a liquid by activating the suction device so that the liquid is drawn from the liquid source through the at least one channel, preferably at least two channels, via the distal end towards the suction device.

## Claims

1. A method for pre-cleaning an endoscope, preferably immediately or shortly after use of the endoscope, the method comprising:
- providing an endoscope that comprises
∘ channels for transporting fluids,
∘ connection ports, in particular for connection of fluid hoses,
∘ and a distal end of the endoscope,
wherein each of the channels extends from one of the connection ports to the distal end of the endoscope;
- fluidly connecting the channels to each other at the distal end of the endoscope by arranging a container onto the distal end of the endoscope;
- connecting a suction device, preferably a suction pump, to one of the connection ports;
- connecting at least one liquid source to at least one of the connection ports;
- flushing at least one of the channels with a liquid by activating the suction device so that the liquid is drawn from the liquid source through the at least one channel via the distal end towards the suction device.

2. The method according to the preceding claim,
wherein connecting at least one liquid source to at least one of the connection ports comprises connecting at least one vessel that holds liquid, preferably at least three, in particular four, vessels that hold liquid, to at least one of the connection ports, preferably to at least three, in particular four, of the connection ports.

3. The method according at least one of the preceding claims, comprising:
- connecting closure elements to each of the connection ports to which no liquid source and no suction device is connected.

4. The method according to at least one of the preceding claims, the method comprising:
- flushing at least two of the channels, preferably all of the channels, with liquid by activating the suction device so that liquid is drawn through the channels via the distal end towards the suction device.

5. The method according to at least one of the preceding claims
wherein the channels comprise
- an air channel that extends from an air channel connection port to the distal end of the endoscope,
- a suction channel that extends from a suction channel connection port to the distal end of the endoscope,
- a water channel that extends from a water channel connection port to the distal end of the endoscope,
- and preferably an auxiliary water channel that extends from an auxiliary water channel connection port to the distal end of the endoscope.

6. The method according to at least one of the preceding claims,
wherein the container is arranged onto the distal end of the endoscope in such a manner that the distal end, including the outer surface of the distal end, is arranged entirely inside the container.

7. The method according to at least one of the preceding claims,
wherein the container is filled with liquid and the whole distal end, including the outer surface of the distal end, is in contact with the liquid.

8. The method according to at least one of the preceding claims,
wherein the container is arranged on the distal end of the endoscope in such a manner that the distal end of the endoscope is sealed liquid-tight, and preferably gas-tight, from the environment that surrounds the container.

9. The method according to at least one of the preceding claims,
wherein each of the connection ports is connected to a vessel, except for the connection port that is connected to the suction device.

10. The method according to at least one of the preceding claims,
wherein the suction device is fluidly connected to a suction container that is adapted to receive liquid drawn out of the endoscope by the suction device.

11. The method according to at least one of the preceding claims,
wherein the liquid that is drawn through at least one of the channels is water or a disinfectant solution or a detergent solution.

12. The method according to at least one of the preceding claims,
wherein the endoscope is immersed in a liquid so that the connection ports are in contact with the liquid, wherein preferably the connection port that is connected to the suction device is connected to the suction device via a hose or the like.

13. An arrangement for pre-cleaning an endoscope, preferably immediately or shortly after use of the endoscope, in particular for pre-cleaning an endoscope according to the method according to at least one of the preceding claims, the arrangement comprising:
- an endoscope that comprises
∘ channels for transporting fluids,
∘ connection ports, in particular for connection of fluid hoses,
∘ and a distal end of the endoscope,
wherein each of the channels extends from one of the connection ports to the distal end of the endoscope;
- a container that is arranged onto the distal end of the endoscope in such a manner that the channels are fluidly connected to each other at the distal end of the endoscope;
- at least one liquid source that is connected to at least one of the connection ports;
- and preferably a suction device, preferably a suction pump, that is connected to one of the connection ports.

14. The arrangement according to the preceding claim,
wherein the suction device is adapted to flush at least one of the channels with a liquid by activating the suction device so that the liquid is drawn from the liquid source through the at least one channel via the distal end towards the suction device.

15. The arrangement according to at least one of the preceding claims, wherein the endoscope comprises:
- an air channel that extends from an air channel connection port to the distal end of the endoscope,
- a suction channel that extends from a suction channel connection port to the distal end of the endoscope,
- a water channel that extends from a water channel connection port to the distal end of the endoscope,
- and preferably an auxiliary water channel that extends from an auxiliary water channel connection port to the distal end of the endoscope,
- and preferably at least one closure element that is connected to each of the connection ports to which no liquid source and no suction device is connected.
